(19) Europäisches Patentamt
European Patent Office
Office européen des brevets

(11) Veröffentlichungsnummer: **0 078 002**
**B1**

(12) # EUROPÄISCHE PATENTSCHRIFT

(45) Veröffentlichungstag der Patentschrift: **21.08.85**

(51) Int. Cl.⁴: **A 01 N 43/90** // C07D513/04

(21) Anmeldenummer: **82109664.1**

(22) Anmeldetag: **20.10.82**

(54) **Herbizide, enthaltend Thiazolo(2,3-b)-chinazolone als Wirkstoffe, und ihre Verwendung zur Beeinflussung des Pflanzenwachstums.**

(30) Priorität: **28.10.81 DE 3142728**

(43) Veröffentlichungstag der Anmeldung: **04.05.83 Patentblatt 83/18**

(45) Bekanntmachung des Hinweises auf die Patenterteilung: **21.08.85 Patentblatt 85/34**

(84) Benannte Vertragsstaaten: **AT BE CH DE FR GB IT LI NL**

(56) Entgegenhaltungen:
**EP - A - 0 006 114**
**EP - A - 0 027 268**
**DE - A - 2 557 425**

**CENTRAL PATENTS INDEX, Basic Abstracts Journal, Section C: Agdoc, Woche B08, 18. April 1979, Zusammenfassung Nr. 14891B/08, London, G.B.**

(73) Patentinhaber: **BASF Aktiengesellschaft, Carl-Bosch-Strasse 38, D-6700 Ludwigshafen (DE)**

(72) Erfinder: **Seybold, Guenther, Dr., Friedrich-Ebert-Strasse 14, D-6708 Neuhofen (DE)**
Erfinder: **Wuerzer, Bruno, Dr. Dipl.-Landw., Ruedigerstrasse 13, D-6701 Otterstadt (DE)**

**EP 0 078 002 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Herbizide und Mittel zur Beeinflussung des Pflanzenwachstums, die Thiazolo[2,3-b]-chinazolone als Wirkstoffe enthalten, sowie Verfahren zur Beeinflussung und Bekämpfung von Pflanzenwuchs mit diesen Herbiziden.

Thiazolo[2,3-b]-chinazolone mit antibakterieller und pharmazeutischer Wirkung sind bekannt (J. Sci. Ind. Research (1958) 17 B, 120—123; DE-OS 2 557 425).

Es wurde gefunden, daß herbizide Mittel, die Thiazolo[2,3-b]-chinazolone der Formel

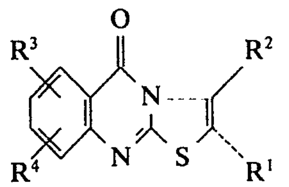

(I)

in der

$R^1$ und $R^2$ unabhängig voneinander Wasserstoff, Halogen, Nitro, Alkyl mit 1 bis 12 Kohlenstoffatomen, Halogenalkyl mit 1 bis 4 Kohlenstoffatomen, Cycloalkyl mit 3 bis 6 Kohlenstoffatomen, Alkoxy oder Alkylthio mit 1 bis 4 Kohlenstoffatomen, Thiophenyl, Amino, Alkylamino oder Dialkylamino mit 1 bis 4 Kohlenstoffatomen in einer Alkylgruppe, Aminoalkyl mit 1 bis 4 Kohlenstoffatomen, Alkylaminoalkyl oder Dialkylaminoalkyl mit 1 bis 4 Kohlenstoffatomen in einer Alkylgruppe, Alkanoylamino oder Halogenalkanoylamino mit 2 bis 5 Kohlenstoffatomen, Dialkylcarbamido mit 1 bis 4 Kohlenstoffatomen in einer Alkylgruppe, Alkoxycarbonylalkyl mit 3 bis 6 Kohlenstoffatomen, gegebenenfalls durch Alkoxy mit 1 bis 4 Kohlenstoffatomen substituiertes Alkoxycarbonyl mit 2 bis 6 Kohlenstoffatomen, Sulfo, Sulfino, Alkylsulfonyl oder Alkylsulfinyl mit 1 bis 4 Kohlenstoffatomen, Chlorsulfonyl, Phenylsulfonyl Sulfamoyl, Alkylaminosulfinyl oder Alkylaminosulfonyl mit 1 bis 4 Kohlenstoffatomen, Hydroxyalkylaminosulfonyl oder Di-hydroxyalkylaminosulfonyl mit 1 bis 4 Kohlenstoffatomen in einer Alkylgruppe, Morpholinyl-sulfonyl, Alkylsulfonylamino mit 1 bis 4 Kohlenstoffatomen, Alkoxycarbonylalkylamino mit 3 bis 6 Kohlenstoffatomen, gegebenenfalls durch Halogen ein- oder zweifach, durch Alkoxy oder Carboxyalkyloxy mit jeweils bis zu 5 Kohlenstoffatomen einfach substituiertes Phenyl oder gegebenenfalls durch Halogen oder Alkyl mit 1 bis 4 Kohlenstoffatomen einfach substituiertes Thien-2-yl, Thiazol-2-yl, Fur-2-yl, Benzimidazol-2-yl, 2-Chlor-thien-4-yl, 2-Methyl-thien-4-yl oder 1-Methyl-benzimidazolyl und

$R^3$ und $R^4$ unabhängig voneinander Wasserstoff, Halogen, Nitro, Alkyl oder Halogenalkyl mit 1 bis 4 Kohlenstoffatomen, Cycloalkyl mit 3 bis 6 Kohlenstoffatomen, Alkoxy oder Alkylthio mit 1 bis 4 Kohlenstoffatomen, Thiophenyl, Amino, Alkylamino oder Dialkylamino mit 1 bis 4 Kohlenstoffatomen in einer Alkylgruppe, Aminoalkyl mit 1 bis 4 Kohlenstoffatomen, Alkylaminoalkyl oder Dialkylaminoalkyl mit 1 bis 4 Kohlenstoffatomen in einer Alkylgruppe, Alkanoylamino oder Halogenalkanoylamino mit 2 bis 5 Kohlenstoffatomen, Dialkylcarbamido mit 1 bis 4 Kohlenstoffatomen in einer Alkylgruppe, Alkoxycarbonylalkyl mit 3 bis 6 Kohlenstoffatomen, gegebenenfalls durch Alkoxy mit 1 bis 4 Kohlenstoffatomen substituiertes Alkoxycarbonyl mit 2 bis 6 Kohlenstoffatomen, Sulfo, Sulfino, Alkylsulfonyl oder Alkylsulfinyl mit 1 bis 4 Kohlenstoffatomen, Chlorsulfonyl, Phenylsulfonyl, Sulfamoyl, Alkylaminosulfinyl oder Alkylaminosulfonyl mit 1 bis 4 Kohlenstoffatomen, Hydroxyalkylaminosulfonyl oder Dihydroxyalkylaminosulfonyl mit 1 bis 4 Kohlenstoffatomen in einer Alkylgruppe, Morpholinyl-sulfonyl, Alkylsulfonylamino mit 1 bis 4 Kohlenstoffatomen, Alkoxycarbonylalkylamino mit 3 bis 6 Kohlenstoffatomen gegebenenfalls durch Halogen ein- oder zweifach, durch Alkoxy oder Carboxyalkyloxy mit jeweils bis zu 5 Kohlenstoffatomen einfach substituiertes Phenyl oder gegebenenfalls durch Halogen oder Alkyl mit 1 bis 4 Kohlenstoffatomen einfach substituiertes Thien-2-yl, Thiazol-2-yl, Fur-2-yl, Benzimidazol-2-yl, 2-Chlor-thien-4-yl, 2-Methylthien-4-yl oder 1-Methyl-benzimidazolyl bedeuten, oder deren Säureadditionssalze enthalten, sehr gut wirksam sind und wachstumsbeeinflussende Eigenschaften haben.

Die Substituenten $R^1$, $R^2$, $R^3$ und $R^4$ in Formel I stehen für Wasserstoff, Halogen, wie Fluor, Chlor, Brom, Jod, Nitro, Alkylreste mit 1 bis 12, vorzugsweise 1 bis 4 Kohlenstoffatomen, wie Methyl, Ethyl, n-Propyl, Isopropyl, n-Butyl, i-Butyl, sec-Butyl, tert.-Butyl, Halogenalkylreste mit 1 bis 4 Kohlenstoffatomen, wie Chlormethyl, Fluormethyl, Difluormethyl, Trichlormethyl, Trifluormethyl, Pentafluorethyl, 2-Chlor-1,1,2,2-tetrafluorethyl, Nonafluor-n-butyl, Cycloalkylreste mit 3 bis 6 Kohlenstoffatomen, wie Cyclopropyl, Cyclopentyl, Cyclohexyl, Alkoxy- oder Alkylthioreste mit 1 bis 4 Kohlenstoffatomen, wie Methoxy, Methylthio, Ethylthio, n-Propylthio, Ethoxy, n-Butoxy, n-Butylthio, Isopropoxy, tert.-Butoxy, Thiophenyl, Amino, Alkyl- oder Dialkylamino mit 1 bis 4 Kohlenstoffatomen in einer Alkylgruppe, wie Methylamino, Dimethylamino, n-Butylamino, Diethylamino, Isopropylamino, Methylethylamino, Aminoalkyl, Alkylaminoalkyl oder Dialkylamioalkyl mit jeweils 1 bis 4 Kohlenstoffatomen in einer Alkylgruppe, wie Aminomethyl, Methylaminomethyl, Dimethylaminomethyl, Aminoethyl, Methylaminoethyl, Dimethylaminoethyl, Alkanoylamino- oder Halogenalkanoylamino mit 2 bis 5 Kohlenstoffatomen,

wie Acetylamino, Chloracetylamino, Trifluoracetylamino, Propionylamino, 2-Chlorpropionylamino, Dialkylcarbamido mit 1 bis 4 Kohlenstoffatomen in einer Alkylgruppe, wie N,N-Dimethylcarbamido, N,N-Diethylcarbamido, N,N-Di-n-butylcarbamido, Alkoxycarbonylalkyl mit 3 bis 6 Kohlenstoffatomen, wie Methoxycarbonylmethyl, Ethoxycarbonylmethyl, n-Butoxycarbonylmethyl, gegebenenfalls durch Alkoxy substituiertes Alkoxycarbonyl mit 2 bis 6 Kohlenstoffatomen, wie Methoxycarbonyl, Ethoxycarbonyl, n-Propoxycarbonyl, n-Butoxycarbonyl, Isopropoxycarbonyl, 2-Methoxyethoxycarbonyl, Ethoxymethoxycarbonyl, 2-Ethoxy-ethoxycarbonyl, Sulfo, Sulfino, Alkylsulfonyl oder Alkylsulfinyl mit 1 bis 4 Kohlenstoffatomen, wie Methylsulfonyl, Methylsulfinyl, Isopropylsulfonyl, n-Propylsulfinyl, n-Butylsulfonyl, n-Butylsulfinyl, Chlorsulfonyl, Phenylsulfonyl, Sulfamoyl, Alkylaminosulfonyl oder Alkylaminosulfinyl mit 1 bis 4 Kohlenstoffatomen, wie Methylaminosulfonyl, Methylaminosulfinyl, Isopropylaminosulfonyl, n-Butylaminosulfonyl, n-Butylaminosulfinyl, Hydroxyalkylamino- oder Di-hydroxyalkylaminosulfonyl mit 1 bis 4 Kohlenstoffatomen in einer Hydroxyalkylgruppe, wie 2-Hydroxyethylaminosulfonyl, Di-(2-hydroxyethyl)aminosulfonyl, Alkylsulfonylamino mit 1 bis 4 Kohlenstoffatomen, wie Methylsulfonylamino, Isopropylsulfonylamino, Isobutylsulfonylamino, Alkoxycarbonylalkylamino mit 3 bis 6 Kohlenstoffatomen, wie 2-Methoxycarbonylethylamino, 2-n-Butoxycarbonylethylamino, gegebenenfalls durch Halogen, Alkoxy oder Carboxyalkyloxy mit jeweils bis zu 5 Kohlenstoffatomen substituiertes Phenyl, wie Phenyl, 4-Chlorphenyl, 4-Methoxyphenyl, 4-Carboxymethoxyphenyl, 4-(2-Carboxyethoxy)-phenyl, 3,5-Dichlorphenyl, oder für gegebenenfalls durch Halogen oder Alkyl mit bis zu 4 Kohlenstoffatomen substituiertes Hetaryl, wie Thien-2-yl, Thiazol-2-yl, Fur-2-yl, Benzimidazol-2-yl, 2-Chlor-thien-4-yl, 2-Methyl-thien-4-yl, 1-Methyl-benzimidazol-yl.

Bevorzugte Verbindungen der Formel I sind solche, bei denen $R^1$ und $R^2$ unabhängig voneinander Wasserstoff, Alkyl oder Halogenalkyl mit jeweils 1 bis 4 Kohlenstoffatomen, Aminoalkyl, Alkylaminoalkyl oder Dialkylaminoalkyl mit jeweils 1 bis 4 Kohlenstoffatomen in einer Alkylgruppe, oder gegebenenfalls durch Chlor, Fluor, Alkoxy mit 1 bis 4 Kohlenstoffatomen oder Alkoxycarbonyl mit 2 bis 5 Kohlenstoffatomen substituiertes Phenyl und $R^3$ und $R^4$ unabhängig voneinander Wasserstoff, Halogen, Alkoxy mit 1 bis 4 Kohlenstoffatomen bedeuten, oder solche, bei denen $R^1$ und $R^2$ unabhängig, voneinander Wasserstoff, Methyl, Ethyl, Brommethyl, Dialkylaminomethyl mit 1 bis 4 Kohlenstoffatomen in einer Alkylgruppe, oder gegebenenfalls durch Chlor, Fluor oder Alkoxy mit 1 bis 4 Kohlenstoffatomen substituiertes Phenyl und $R^3$ und $R^4$ unabhängig voneinander Wasserstoff, Chlor, Brom, Methoxy, bedeuten, oder solche, bei denen $R^1$ Wasserstoff, Methyl, Ethyl oder Brommethyl, $R^2$ Wasserstoff, Methyl oder Ethyl und $R^3$ und $R^4$ unabhängig voneinander Wasserstoff, Chlor oder Brom bedeuten, oder Säureadditionssalze dieser Verbindungen. Bei den zuletzt genannten Verbindungen stehen die Substituenten Chlor und Brom für $R^3$ und $R^4$ vorzugsweise in 7- und/oder 9-Stellung.

Insbesondere bevorzugt sind 3-Methyl-thiazolo[2,3-b]-chinazolone, die in 2-Stellung unsubstituiert sind oder einen Methyl-Substituenten tragen, und Säureadditionssalze dieser Verbindungen.

Man erhält die Thiazolo[2,3-b]-chinazolone der Formel I durch Umsetzung von Anthranilsäurederivaten der Formel

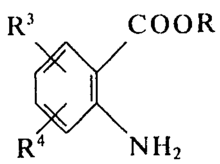

(II)

in der
R Wasserstoff oder Alkyl, vorzugsweise mit 1 bis 4 Kohlenstoffatomen, bedeutet und $R^3$ und $R^4$ die oben genannten Bedeutungen haben,
mit einem Thiocyanatderivat der Formel

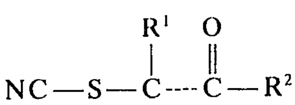

(III)

in der
$R^1$ und $R^2$ die obengenannten Bedeutungen haben, oder einem Thiazolderivat der Formel

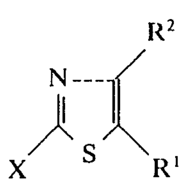

(IV)

in der
X Fluor, Chlor, Brom, Alkylsulfonyl oder Arylsulfonyl bedeutet und $R^1$ und $R^2$ die obengenannten Bedeutungen haben.

Die Kondensation des Anthranilsäurederivats der Formel II mit dem Thiocyanatderivat der Formel III wird vorzugsweise in wäßrigem Medium in Gegenwart starker Mineralsäuren, wie Chlorwasserstoff, Bromwasserstoff oder Schwefelsäure, durchgeführt. Die Ausgangsstoffe werden vorzugsweise in ungefähr stöchiometrischem Verhältnis eingesetzt; ein Überschuß der einen oder anderen Reaktionskomponente beeinträchtigt den Reaktionsablauf nicht. Die Reaktionstemperatur kann im Bereich zwischen 40 und 150°C, vorzugsweise zwischen 60 und 100°C, variiert werden. Die Säuremenge beträgt 1 bis 2 Moläquivalente, bezogen auf die Menge an Anthranilsäurederivat der Formel II.

Die Umsetzung des Anthranilsäurederivats der Formel II mit dem Thiazolderivat der Formel IV wird bei einer Temperatur im Bereich zwischen 100 und 200°C, vorzugsweise zwischen 130 und 160°C, durchgeführt. Die Reaktionskomponenten werden dabei ebenfalls in ungefähr stöchiometrischem Verhältnis eingesetzt. Ein Säurezusatz ist nicht erforderlich. Die Umsetzung kann gegebenenfalls in Gegenwart eines Lösungsmittels durchgeführt werden. In Betracht kommen halogenierte Kohlenwasserstoffe, wie Dichlorbenzol, Trichlorbenzol, mehrwertige Alkohole, wie Glykol, Ethylglykol, Butylglykol, Diethylenglykol, Ester, wie Methylglykolacetat und Dimethylformamid. Auch Gemische dieser Lösungsmittel können verwendet werden. Vorzugsweise führt man die Umsetzung lösungsmittelfrei durch. X in Formel IV bedeutet vorzugsweise Chlor.

Die als Ausgangsstoffe eingesetzten Anthranilsäurederivate der Formel II, Thiocyanatderivate der Formel III und Thiozolderivate der Formel IV sind zum Teil bekannt; sie können in Analogie zu bekannten Methoden hergestellt werden (JACS 74, 1719 (1952); Proc. Ind. Acad. Sci. 22A, 343 (1945)).

Die Säureadditionssalze der Thiazolo[2,3-b]-chinazolone der Formel I erhält man durch Protonierung mit den entsprechenden Säuren in Gegenwart eines inerten Lösungsmittels, wie Tetrahydrofuran, Dioxan, tert.-Butyl-methylether, Methylenchlorid oder Acetonitril.

Als Säuren, die entsprechende Salze bilden, kommen anorganische Säuren, wie Chlorwasserstoffsäure, Bromwasserstoffsäure, Fluorwasserstoffsäure, Schwefelsäure, Phosphorsäure, Perchlorsäure, oder organische Säuren, wie Monochloressigsäure, Dichloressigsäure, Trichloressigsäure, Trifluoressigsäure, in Betracht.

## Beispiel

389 Teile Anthranilsäure werden in 3000 Teilen 5%iger Salzsäure vorgelegt. Bei 80°C läßt man innerhalb einer Stunde 320 Teile Acetylmethylthiocyanat zutropfen, erhitzt 3 Stunden unter Rückfluß und saugt das Produkt in der Kälte ab. Man erhält 422 Teile 3-Methyl-5H-thiazolo[2,3-b]-chinazolin-5-on vom Schmelzpunkt 180°C.

20 Teile 3-Methyl-5H-thiazolo[2,3-b]-chinazolin-5-on werden bei 50°C in 150 Teilen Tetrahydrofuran gelöst und portionsweise mit 34 Teilen Bromwasserstoffsäure versetzt. Das Reaktionsprodukt wird abgesaugt, mit Aceton gewaschen und getrocknet. Man erhält 26,4 Teile des Hydrobromids vom Schmelzpunkt 300—305°C.

Analyse:
Berechnet: Br 26,9
Gefunden: Br 27,0.

Thiazolo[2,3-b]-chinazolone der Formel I, die als Wirkstoffe in den erfindungsgemäßen Herbiziden enthalten sein können, sind beispielsweise:

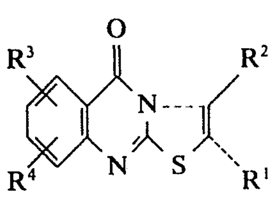

| Wirkstoff Nr. | $R^1$ | $R^2$ | $R^3$ | $R^4$ | Schmp. [°C] |
|---|---|---|---|---|---|
| 1 | H | $CH_3$ | H | H | 180 |
| 2 | H | $CH_3$ | H | H (Perchlorat) | 310—315 |
| 3 | H | $CH_3$ | H | H (Sulfat) | 315—320 |
| 4 | $CH_3$ | $CH_3$ | H | H | 175 |
| 5 | $CH_3$ | $CH_3$ | H | H (Hydrobromid) | 300—310 |
| 6 | Br | $CH_3$ | H | H | 300—305 |
| 7 | H | H | H | H | 144—146 |
| 8 | H | H | H | H (Hydrobromid) | 308—310 |
| 9 | H | $CH_3$ | 7-$CH_3$ | H | 180 |
| 10 | $CH_3$ | H | 8-$CH_3$ | H | 165 |
| 11 | H | 4-Chlorphenyl | 8-$CH_3$ | H | 220 |
| 12 | $CH_2Br$ | H | H | H | 210 |
| 13 | $CH_3$ | H | 6-$CH_3$ | H | 168 |
| 14 | $CH_3$ | H | H | H | 183 |
| 15 | $CH_3$ | H | 7-$CH_3$ | H | 200 |

Die erfindungsgemäßen Herbizide werden in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Poly-

oxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die herbiziden Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. Man vermischt 90 Gewichtsteile der Verbindung Nr. 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 7 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteile des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile der Verbindung Nr. 8 werden in einer Mischung gelöst, die aus 25 Gewichtsteilen Cyclohexanol, 65 Gewichtsteilen einer Mineralölfraktion vom Siedepunkt 210 bis 280° C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichtsteile der Verbindung Nr. 4 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile der Verbindung Nr. 3 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile der Verbindung Nr. 6 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile der Verbindung Nr. 6 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation der herbiziden Mittel kann im Vorauflaufverfahren oder im Nachauflaufverfahren, vorzugsweise im Nachauflaufverfahren, erfolgen. Dabei können die Mittel auf den Standort aufgebracht werden, bevor die unerwünschten Pflanzen aus den Samen gekeimt oder aus vegetativen Pflanzenteilen ausgetrieben haben, oder sie werden auf die Blätter der unerwünschten Pflanzen und der Kulturpflanzen appliziert. Vorzugsweise werden die neuen Herbizide nach dem Auflaufen der unerwünschten Pflanzen, sowohl auf Kulturflächen als auch auf unbebautem Land, ausgebracht. Sind die Wirkstoffe für die Kulturpflanze weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit und Wachstumsstadium 0,05 bis

15 kg/ha und mehr, vorzugsweise 0,125 bis 5 kg/ha, wobei sich die höheren Dosen besonders zur totalen Bekämpfung von Vegetationen eignen.

Die herbizide und wachstumsbeeinflussende Wirkung von herbiziden Mitteln, die Verbindungen der Formel I enthalten, wird durch Gewächshausversuche gezeigt:

Als Kulturgefäße dienen Plastikblumentöpfe mit 300 $m^3$ Inhalt und lehmigem Sand mit etwa 1,5% Humus als Substrat. Bei dem für die Nachauflaufbehandlung angezogenen Reis wird Torfmull zugesetzt, um ein einwandfreies Wachstum zu gewährleisten. Die Samen der Testpflanzen werden nach Arten getrennt flach eingesät. Unmittelbar danach erfolgt bei Vorauflaufbehandlung das Aufbringen der Wirkstoffe auf die Erdoberfläche. Sie werden hierzu in Wasser als Verteilungsmittel suspendiert oder emulgiert und mittels fein verteilender Düsen gespritzt. Die Aufwandmengen können rund 2 kg Wirkstoff pro ha betragen. Nach dem Aufbringen der Mittel beregnet man die Gefäße leicht, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testplanzen, sofern dies nicht durch die Wirkstoffe beeinträchtigt wird.

Zur Nachauflaufanwendung werden entweder direkt gesäte und in den gleichen Gefäßen aufgewachsene Pflanzen oder zunächst als Keimpflanzen getrennt angezogene und einige Tage vor der Behandlung in die Versuchsgefäße verpflanzte Pflanzen verwendet. Je nach Wuchsform haben die Pflanzen zum Behandlungstermin eine Höhe von etwa 3 bis 15 cm. Die Aufwandmengen variieren je nach Wirkstoff und liegen beispielsweise bei 0,5, 1,0 oder 3,0 kg Wirkstoff/ha im Nachauflaufverfahren.

Die Versuchsgefäße werden im Gewächshaus aufgestellt, wobei für wärmeliebende Arten wärmere Bereiche (20 bis 30° C) und für solche gemäßigter Klimate 15 bis 25° C bevorzugt werden. Die Versuchsperiode erstreckt sich über 3 bis 4 Wochen. Während dieser Zeit werden die Pflanzen gepflegt, und ihre Reaktion wird auf die einzelnen Behandlungen ausgewertet. Bewertet wird nach einer Skala von 0 bis 100. Dabei bedeutet 0 keine Schädigung oder normaler Ablauf und 100 kein Aufgang der Pflanzen bzw. völlige Zerstörung zumindest der oberirdischen Teile.

Bei den Testpflanzen handelt es sich um Abutilon theophrasti (Chinesischer Hanf), Acanthospermum hispidum, Amaranthus spp. (Fuchsschwanz), Arachis hypogaea (Erdnüsse), Avena sativa (Hafer), Chenopodium album (Weißer Gänsefuß), Desmodium tortuosum, Euphorbia geniculata (Südamerik. Wolfsmilchart), Lamium purpureum (Rote Taubnessel), Mercurialis annua (Einjähriges Bingelkraut), Nicandra physaloides (Giftbeere), Oryza sativa (Reis), Rumex obtusifolium (Stumpfblättriger Ampfer), Sida spinosa, Solanum nigrum (Schwarzer Nachtschatten), Thlaspi arvense (Acker-Hellerkraut), Triticum aestivum (Weizen), Veronica persica (Persischer Ehrenpreis), Zea mays (Mais), Avena sativum (Hafer), Daucus carota (Möhre), Gossypium hirsutum (Baumwolle), Hordeum vulgare (Gerste), Setaria spp. (Borstenhirsearten).

Bei der Prüfung auf selektive herbizide Wirkung bei Nachauflaufanwendung zeigen erfindungsgemäße Herbizide, die die Verbindungen Nr. 1, 2, 3, 4, 6, 7 und 8 enthalten, eine gute herbizide Wirkung. Die Aufwandmengen schwanken dabei zwischen 0,5 und 3,0 kg Wirkstoff/ha.

Bei Vorauflaufanwendung bekämpfen beispielsweise die Verbindungen Nr. 1, 2, 3, 6 und 7 unerwünschte Pflanzen mit 2,0 kg Wirkstoff/ha, ohne Getreidearten zu schädigen.

In Anbetracht der Verträglichkeit der Wirkstoffe und der Vielseitigkeit der Applikationsmethoden können die erfindungsgemäßen Mittel außer bei den in den Gewächshausversuchen getesteten Nutzpflanzen noch in einer weiteren großen Zahl von Kulturpflanzen zur Beseitigung unerwünschten Pflanzenwuchses eingesetzt werden.

In Betracht kommen beispielsweise die folgenden Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |

(Fortsetzung)

| Botanischer Name | Deutscher Name |
|---|---|
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor – Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |

(Fortsetzung)

| Botanischer Name | Deutscher Name |
|---|---|
| Linum usitatissimum | Faserlein |
| Lycopersiocon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- u. Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Urdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |

(Fortsetzung)

| Botanischer Name | Deutscher Name |
|---|---|
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium carymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die erfindungsgemäßen Mittel mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem ist es nützlich, die erfindungsgemäßen Mittel allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Zur Aktivierung der herbiziden Wirkung können auch Netz- und Haftmittel sowie nicht-phytotoxische Öle und Ölkonzentrate zugesetzt werden.

## Patentansprüche

1. Herbizid, enthaltend ein Thiazolo[2,3-b]-chinazolon der Formel

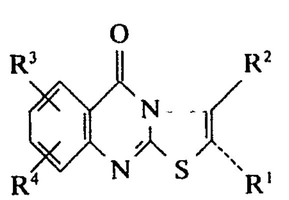

(I)

in der

$R^1$ und $R^2$ unabhängig voneinander Wasserstoff, Halogen, Nitro, Alkyl mit 1 bis 12 Kohlenstoffatomen, Halogenalkyl mit 1 bis 4 Kohlenstoffatomen, Cycloalkyl mit 3 bis 6 Kohlenstoffatomen, Alkoxy oder Alkylthio mit 1 bis 4 Kohlenstoffatomen, Thiophenyl, Amino, Alkylamino oder Dialkylamino mit 1 bis 4 Kohlenstoffatomen in einer Alkylgruppe, Aminoalkyl mit 1 bis 4 Kohlenstoffatomen, Alkylaminoalkyl

oder Dialkylaminoalkyl mit 1 bis 4 Kohlenstoffatomen in einer Alkylgruppe, Alkanoylamino oder Halogenalkanoylamino mit 2 bis 5 Kohlenstoffatomen, Dialkylcarbamido mit 1 bis 4 Kohlenstoffatomen in einer Alkylgruppe, Alkoxycarbonylalkyl mit 3 bis 6 Kohlenstoffatomen, gegebenenfalls durch Alkoxy mit 1 bis 4 Kohlenstoffatomen substituiertes Alkoxycarbonyl mit 2 bis 6 Kohlenstoffatomen, Sulfo, Sulfino, Alkylsulfonyl oder Alkylsulfinyl mit 1 bis 4 Kohlenstoffatomen, Chlorsulfonyl, Phenylsulfonyl, Sulfamoyl, Alkylaminosulfinyl oder Alkylaminosulfonyl mit 1 bis 4 Kohlenstoffatomen, Hydroxyalkylaminosulfonyl oder Di-hydroxyalkylaminosulfonyl mit 1 bis 4 Kohlenstoffatomen in einer Alkylgruppe, Morpholinyl-sulfonyl, Alkylsulfonylamino mit 1 bis 4 Kohlenstoffatomen, Alkoxycarbonylalkylamino mit 3 bis 6 Kohlenstoffatomen, gegebenenfalls durch Halogen ein- oder zweifach, durch Alkoxy oder Carboxyalkyloxy mit jeweils bis zu 5 Kohlenstoffatomen einfach substituiertes Phenyl oder gegebenenfalls durch Halogen oder Alkyl mit 1 bis 4 Kohlenstoffatomen einfach substituiertes Thien-2-yl, Thiazol 2-yl, Fur-2-yl, Benzimidazol-2-yl, 2-Chlor-thien-4-yl, 2-Methyl-thien-4-yl oder 1-Methyl-benzimidazolyl und

$R^3$ und $R^4$ unabhängig voneinander Wasserstoff, Halogen, Nitro, Alkyl oder Halogenalkyl mit 1 bis 4 Kohlenstoffatomen, Cycloalkyl mit 3 bis 6 Kohlenstoffatomen, Alkoxy oder Alkylthio mit 1 bis 4 Kohlenstoffatomen, Thiophenyl, Amino, Alkylamino oder Dialkylamino mit 1 bis 4 Kohlenstoffatomen in einer Alkylgruppe, Aminoalkyl mit 1 bis 4 Kohlenstoffatomen, Alkylaminoalkyl oder Dialkylaminoalkyl mit 1 bis 4 Kohlenstoffatomen in einer Alkylgruppe, Alkanoylamino oder Halogenalkanoylamino mit 2 bis 5 Kohlenstoffatomen, Dialkylcarbamido mit 1 bis 4 Kohlenstoffatomen in einer Alkylgruppe, Alkoxycarbonylalkyl mit 3 bis 6 Kohlenstoffatomen, gegebenenfalls durch Alkoxy mit 1 bis 4 Kohlenstoffatomen substituiertes Alkoxycarbonyl mit 2 bis 6 Kohlenstoffatomen, Sulfo, Sulfino, Alkylsulfonyl oder Alkylsulfinyl mit 1 bis 4 Kohlenstoffatomen, Chlorsulfonyl, Phenylsulfonyl, Sulfamoyl, Alkylaminosulfinyl oder Alkylaminosulfonyl mit 1 bis 4 Kohlenstoffatomen, Hydroxyalkylaminosulfonyl oder Dihydroxyalkylaminosulfonyl mit 1 bis 4 Kohlenstoffatomen in einer Alkylgruppe, Morpholinyl-sulfonyl, Alkylsulfonylamino mit 1 bis 4 Kohlenstoffatomen, Alkoxycarbonylalkylamino mit 3 bis 6 Kohlenstoffatomen gegebenenfalls durch Halogen ein- oder zweifach, durch Alkoxy oder Carboxyalkyloxy mit jeweils bis zu 5 Kohlenstoffatomen einfach substituiertes Phenyl oder gegebenenfalls durch Halogen oder Alkyl mit 1 bis 4 Kohlenstoffatomen einfach substituiertes Thien-2-yl, Thiazol-2-yl, Fur-2-yl, Benzimidazol-2-yl, 2-Chlor-thien-4-yl, 2-Methylthien-4-yl oder 1-Methyl-benzimidazolyl bedeuten, oder Säureadditionssalze dieser Verbindung.

2. Herbizid, enthaltend inerte Zusatzstoffe und ein Thiazolo[2,3-b]-chinazolon der Formel

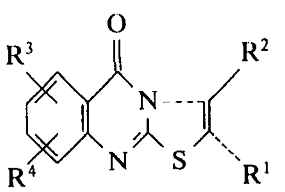

(I)

in der

$R^1$ und $R^2$ unabhängig voneinander Wasserstoff, Halogen, Nitro, Alkyl mit 1 bis 12 Kohlenstoffatomen, Halogenalkyl mit 1 bis 4 Kohlenstoffatomen, Cycloalkyl mit 3 bis 6 Kohlenstoffatomen, Alkoxy oder Alkylthio mit 1 bis 4 Kohlenstoffatomen, Thiophenyl, Amino, Alkylamino oder Dialkylamino mit 1 bis 4 Kohlenstoffatomen in einer Alkylgruppe, Aminoalkyl mit 1 bis 4 Kohlenstoffatomen, Alkylaminoalkyl oder Dialkylaminoalkyl mit 1 bis 4 Kohlenstoffatomen in einer Alkylgruppe, Alkanoylamino oder Halogenalkanoylamino mit 2 bis 5 Kohlenstoffatomen, Dialkylcarbamido mit 1 bis 4 Kohlenstoffatomen in einer Alkylgruppe, Alkoxycarbonylalkyl mit 3 bis 6 Kohlenstoffatomen, gegebenenfalls durch Alkoxy mit 1 bis 4 Kohlenstoffatomen substituiertes Alkoxycarbonyl mit 2 bis 6 Kohlenstoffatomen, Sulfo, Sulfino, Alkylsulfonyl oder Alkylsulfinyl mit 1 bis 4 Kohlenstoffatomen, Chlorsulfonyl, Phenylsulfonyl, Sulfamoyl, Alkylaminosulfinyl oder Alkylaminosulfonyl mit 1 bis 4 Kohlenstoffatomen, Hydroxyalkylaminosulfonyl oder Di-hydroxyalkylaminosulfonyl mit 1 bis 4 Kohlenstoffatomen in einer Alkylgruppe, Morpholinyl-sulfonyl, Alkylsulfonylamino mit 1 bis 4 Kohlenstoffatomen, Alkoxycarbonylalkylamino mit 3 bis 6 Kohlenstoffatomen, gegebenenfalls durch Halogen ein- oder zweifach, durch Alkoxy oder Carboxyalkyloxy mit jeweils bis zu 5 Kohlenstoffatomen einfach substituiertes Phenyl oder gegebenenfalls durch Halogen oder Alkyl mit 1 bis 4 Kohlenstoffatomen einfach substituiertes Thien-2-yl, Thiazol-2-yl, Fur-2-yl, Benzimidazol-2-yl, 2-Chlor-thien-4-yl, 2-Methyl-thien-4-yl oder 1-Methyl-benzimidazolyl und

$R^3$ und $R^4$ unabhängig voneinander Wasserstoff, Halogen, Nitro, Alkyl oder Halogenalkyl mit 1 bis 4 Kohlenstoffatomen, Cycloalkyl mit 3 bis 6 Kohlenstoffatomen, Alkoxy oder Alkylthio mit 1 bis 4 Kohlenstoffatomen, Thiophenyl, Amino, Alkylamino oder Dialkylamino mit 1 bis 4 Kohlenstoffatomen in einer Alkylgruppe, Aminoalkyl mit 1 bis 4 Kohlenstoffatomen, Alkylaminoalkyl oder Dialkylaminoalkyl mit 1 bis 4 Kohlenstoffatomen in einer Alkylgruppe, Alkanoylamino oder Halogenalkanoylamino mit 2 bis 5 Kohlenstoffatomen, Dialkylcarbamido mit 1 bis 4 Kohlenstoffatomen in einer Alkylgruppe, Alkoxycarbonylalkyl mit 3 bis 6 Kohlenstoffatomen, gegebenenfalls durch Alkoxy mit 1 bis 4 Kohlenstoffatomen substituiertes Alkoxycarbonyl mit 2 bis 6 Kohlenstoffatomen, Sulfo, Sulfino, Alkylsulfonyl oder Alkylsulfinyl mit 1 bis 4 Kohlenstoffatomen, Chlorsulfonyl, Phenylsulfonyl, Sulfamoyl, Alkylaminosulfinyl oder Alkylaminosulfonyl mit 1 bis 4 Kohlenstoffatomen, Hydroxyalkylaminosulfonyl oder Dihydroxyalkylaminosulfonyl mit 1 bis 4 Kohlenstoffatomen in einer Alkylgruppe, Morpholinyl-sulfonyl, Alkylsulfonylamino mit 1 bis 4 Kohlenstoffatomen, Alkoxycarbonylalkylamino mit 3 bis 6 Kohlenstoffatomen, gegebenenfalls durch Halogen ein- oder zweifach, durch Alkoxy oder Carboxyalkyloxy mit jeweils bis zu 5 Kohlenstoffatomen einfach substituiertes Phenyl oder gegebenenfalls durch Halogen oder Alkyl mit 1 bis 4 Kohlenstoffatomen einfach substituiertes Thien-2-yl, Thiazol-2-yl, Fur-2-yl, Benzimidazol-2-yl, 2-Chlor-thien-4-yl, 2-Methylthien-4-yl oder 1-Methyl-benzimidazolyl bedeuten,
oder Säureadditionssalze dieser Verbindung.

3. Herbizid, enthaltend ein Thiazolo[2,3-b]-chinazolon der Formel I gemäß Anspruch 1, wobei $R^1$ und $R^2$ unabhängig voneinander Wasserstoff, Alkyl oder Halogenalkyl mit 1 bis 4 Kohlenstoffatomen, Aminoalkyl mit 1 bis 4 Kohlenstoffatomen, Alkylaminoalkyl oder Dialkylaminoalkyl mit 1 bis 4 Kohlenstoffatomen in einer Alkylgruppe oder gegebenenfalls durch Chlor oder Fluor ein- oder zweifach, durch Alkoxy mit 1 bis 4 Kohlenstoffatomen oder Alkoxycarbonyl mit 2 bis 5 Kohlenstoffatomen einfach substituiertes Phenyl und $R^3$ und $R^4$ unabhängig voneinander Wasserstoff, Halogen oder Alkoxy mit 1 bis 4 Kohlenstoffatomen, bedeuten,
oder Säureadditionssalze dieser Verbindung.

4. Herbizid, enthaltend inerte Zusatzstoffe und ein Thiazolo[2,3-b]-chinazolon der Formel I gemäß Anspruch 1, wobei $R^1$ und $R^2$ unabhängig voneinander Wasserstoff, Alkyl oder Halogenalkyl mit 1 bis 4 Kohlenstoffatomen, Aminoalkyl mit 1 bis 4 Kohlenstoffatomen, Alkylaminoalkyl oder Dialkylaminoalkyl mit 1 bis 4 Kohlenstoffatomen in einer Alkylgruppe oder gegebenenfalls durch Chlor oder Fluor ein- oder zweifach, durch Alkoxy mit 1 bis 4 Kohlenstoffatomen oder Alkoxycarbonyl mit 2 bis 5 Kohlenstoffatomen einfach substituiertes Phenyl und $R^3$ und $R^4$ unabhängig voneinander Wasserstoff, Halogen oder Alkoxy mit 1 bis 4 Kohlenstoffatomen, bedeuten,
oder Säureadditionssalze dieser Verbindung.

5. Herbizid, enthaltend ein Thiazolo[2,3-b]-chinazolon der Formel I gemäß Anspruch 1, wobei $R^1$ und $R^2$ unabhängig voneinander Wasserstoff, Methyl, Ethyl, Brommethyl, Dialkylaminometyl mit 1 bis 4 Kohlenstoffatomen oder gegebenenfalls durch Chlor oder Fluor ein oder zweifach, durch Alkoxy mit 1 bis 4 Kohlenstoffatomen einfach substituiertes Phenyl und $R^3$ und $R^4$ unabhängig voneinander Wasserstoff, Chlor, Brom oder Methoxy bedeuten,
oder Säureadditionssalze dieser Verbindung.

6. Herbizid, enthaltend ein Thiazolo[2,3-b]-chinazolon der Formel I gemäß Anspruch 1, wobei $R^1$ Wasserstoff, Methyl, Ethyl oder Brommethyl, $R^2$ Wasserstoff, Methyl oder Ethyl und $R^3$ und $R^4$ unabhängig voneinander Wasserstoff, Chlor oder Brom bedeuten,
oder Säureadditionssalze dieser Verbindung.

7. Herbizid, enthaltend inerte Zusatzstoffe und ein Thiazolo[2,3-b]-chinazolon der Formel I gemäß Anspruch 1, wobei $R^1$ Wasserstoff, Methyl, Ethyl oder Brommethyl, $R^2$ Wasserstoff, Methyl oder Ethyl und $R^3$ und $R^4$ unabhängig voneinander Wasserstoff, Chlor oder Brom bedeuten,
oder Säureadditionssalze dieser Verbindung.

8. Herbizid, enthaltend ein Thiazolo[2,3-b]-chinazolon der Formel

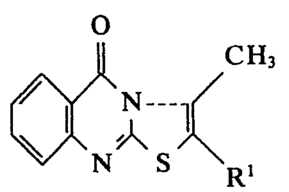

in der
$R^1$ Wasserstoff oder Methyl bedeutet,
oder Säureadditionssalze dieser Verbindung.

9. Herbizid, enthaltend inerte Zusatzstoffe und ein Thiazolo[2,3-b]-chinazolon der Formel

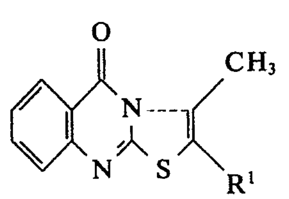

in der
$R^1$ Wasserstoff oder Methyl bedeutet,
oder Säureadditionssalze dieser Verbindung.

10. Verfahren zur Bekämpfung unerwünschten Pflanzenwuchses und zur Beeinflussung des Pflanzenwachstums, dadurch gekennzeichnet, daß man ein Herbizid gemäß Anspruch 1 auf die Pflanzen und/oder ihren Standort einwirken läßt.

**Claims**

1. A herbicide containing a thiazolo[2,3-b]-quinazolone of the formula

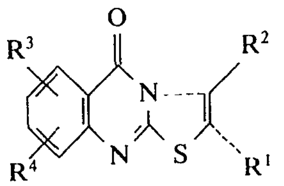

(I)

where
$R^1$ and $R^2$ independently of one another are hydrogen, halogen, nitro, alkyl of 1 to 12 carbon atoms, haloalkyl of 1 to 4 carbon atoms, cycloalkyl of 3 to 6 carbon atoms, alkoxy or alkylthio of 1 to 4 carbon atoms, thiophenyl, amino, alkylamino or dialkylamino where alkyl is of 1 to 4 carbon atoms, aminoalkyl of 1 to 4 carbon atoms, alkylaminoalkyl or dialkylaminoalkyl where alkyl is of 1 to 4 carbon atoms, alkanoylamino or haloalkanoylamino of 2 to 5 carbon atoms, dialkylcarbamido where alkyl is of 1 to 4 carbon atoms, alkoxycarbonylalkyl of 3 to 6 carbon atoms, unsubstituted or $C_1-C_4$-alkoxy-substituted alkoxycarbonyl of 2 to 6 carbon atoms, sulfo, sulfino, alkylsulfonyl or alkylsulfinyl of 1 to 4 carbon atoms, chlorosulfonyl, phenylsulfonyl, sulfamyl, alkylaminosulfinyl or alkylaminosulfonyl of 1 to 4 carbon atoms, hydroxyalkylaminosulfonyl or dihydroxyalkylaminosulfonyl where alkyl is of 1 to 4 carbon atoms, morpholinylsulfonyl, alkylsulfonylamino of 1 to 4 carbon atoms, alkoxycarbonylalkylamino of 3 to 6 carbon atoms, phenyl optionally mono- or disubstituted by halogen or optionally monosubstituted by alkoxy or carboxyalkoxy each having up to 5 carbon atoms, or unsubstituted or halogen- or $C_1-C_4$-alkyl-monosubstituted thien-2-yl, thiazol-2-yl, fur-2-yl, benzimidazol-2-yl, 2-chlorothien-4-yl, 2-methylthien-4-yl or 1-methylbenzimidazolyl, and
$R^3$ and $R^4$ independently of one another are hydrogen, halogen, nitro, alkyl or haloalkyl of 1 to 4 carbon atoms, cycloalkyl of 3 to 6 carbon atoms, alkoxy or alkylthio of 1 to 4 carbon atoms, thiophenyl, amino, alkylamino or dialkylamino of 1 to 4 carbon atoms, aminoalkyl of 1 to 4 carbon atoms, alkylaminoalkyl or dialkylaminoalkyl where alkyl is of 1 to 4 carbon atoms, alkanoylamino or haloalkanoylamino of 2 to 5 carbon atoms, dialkylcarbamido where alkyl is of 1 to 4 carbon atoms, alkoxycarbonylalkyl of 3 to 6 carbon atoms, unsubstituted or $C_1-C_4$-alkoxy-substituted alkoxycarbonyl of 2 to 6 carbon atoms, sulfo, sulfino, alkylsulfonyl or alkylsulfinyl of 1 to 4 carbon atoms, chlorosulfonyl, phenylsulfonyl, sulfamyl, alkylaminosulfinyl or alkylaminosulfonyl of 1 to 4 carbon atoms, hyroxyalkylaminosulfonyl or dihydroxyalkylaminosulfonyl where alkyl is of 1 to 4 carbon atoms, morpholinylsulfonyl, alkylsulfonylamino of 1 to 4 carbon atoms, alkoxycarbonylalkylamino of 3 to 6 carbon atoms, phenyl optionally mono- or disubstituted by halogen or optionally monosubstituted by alkoxy or carboxyalkoxy each having up to 5 carbon atoms, or unsubstituted or halogen- or $C_1-C_4$-alkyl-monosubstituted thien-2-yl, thiazol-2-yl, fur-2-yl, benzimidazol-2-yl, 2-chlorothien-2-yl, 2-methylthien-4-yl or 1-methylbenzimidazolyl,
or an acid addition salt of this compound.

2. A herbicide containing inert additives and a thiazolo-[2,3-b]-quinazolone of the formula

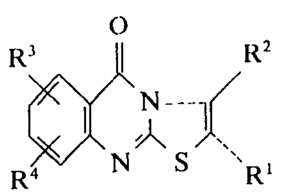

(I)

where
$R^1$ and $R^2$ independently of one another are hydrogen, halogen, nitro, alkyl of 1 to 12 carbon atoms, haloalkyl of 1 to 4 carbon atoms, cycloalkyl of 3 to 6 carbon atoms, alkoxy or alkylthio of 1 to 4 carbon atoms, thiophenyl, amino, alkylamino or dialkylamino where alkyl is of 1 to 4 carbon atoms, aminoalkyl of 1 to 4 carbon atoms, alkylaminoalkyl or dialkylaminoalkyl where alkyl is of 1 to 4 carbon atoms,

alkanoylamino or haloalkanoylamino of 2 to 5 carbon atoms, dialkylcarbamido where alkyl is of 1 to 4 carbon atoms, alkoxycarbonylalkyl of 3 to 6 carbon atoms, unsubstituted or $C_1-C_4$-alkoxy-substituted alkoxycarbonyl of 2 to 6 carbon atoms, sulfo, sulfino, alkylsulfonyl or alkylsulfinyl of 1 to 4 carbon atoms, chlorosulfonyl, phenylsulfonyl, sulfamyl, alkylaminosulfinyl or alkylaminosulfonyl of 1 to 4 carbon atoms, hydroxyalkylaminosulfonyl or dihydroxyalkylaminosulfonyl where alkyl is of 1 to 4 carbon atoms, morpholinylsulfonyl, alkylsulfonylamino of 1 to 4 carbon atoms, alkoxycarbonylalkylamino of 3 to 6 carbon atoms, phenyl optionally mono- or disubstituted by halogen or optionally monosubstituted by alkoxy or carboxyalkoxy each having up to 5 carbon atoms, or unsubstituted or halogen- or $C_1-C_4$-alkyl-monosubstituted thien-2-yl, thiazol-2-yl, fur-2-yl, benzimidazol-2-yl, 2-chlorothien-4-yl, 2-methylthien-4-yl or 1-methylbenzimidazolyl, and

$R^3$ and $R^4$ independently of one another are hydrogen, halogen, nitro, alkyl or haloalkyl of 1 to 4 carbon atoms, cycloalkyl of 3 to 6 carbon atoms, alkoxy or alkylthio ot 1 to 4 carbon atoms, thiophenyl, amino, alkylamino or dialkylamino of 1 to 4 carbon atoms, aminoalkyl of 1 to 4 carbon atoms, alkylaminoalkyl or dialkylaminoalkyl where alkyl is of 1 to 4 carbon atoms, alkanoylamino or haloalkanoylamino of 2 to 5 carbon atoms, dialkylcarbamido where alkyl is of 1 to 4 carbon atoms, alkoxycarbonylalkyl of 3 to 6 carbon atoms, unsubstituted or $C_1-C_4$-alkoxy-substituted alkoxycarbonyl of 2 to 6 carbon atoms, sulfo, sulfino, alkylsulfonyl or alkylsulfinyl of 1 to 4 carbon atoms, chlorosulfonyl, phenylsulfonyl, sulfamyl, alkylaminosulfinyl or alkylaminosulfonyl of 1 to 4 carbon atoms, hydroxyalkylaminosulfonyl or dihydroxyalkylaminosulfonyl where alkyl is of 1 to 4 carbon atoms, morpholinylsulfonyl, alkylsulfonylamino of 1 to 4 carbon atoms, alkoxycarbonylalkylamino of 3 to 6 carbon atoms, phenyl optionally mono- or disubstituted by halogen or optionally monosubstituted by alkoxy or carboxyalkoxy each having up to 5 carbon atoms, or unsubstituted or halogen- or $C_1-C_4$-alkyl-monosubstituted thien-2-yl, thiazol-2-yl, fur-2-yl, benzimidazol-2-yl, 2-chlorothien-2-yl, 2-methylthien-4-yl or 1-methylbenzimidazolyl,

or an acid addition salt of this compound.

3. A herbicide containing a thiazolo[2,3-b]-quinazolone of the formula I as claimed in claim 1, where $R^1$ and $R^2$, independently of one another, are hydrogen, alkyl or haloalkyl of 1 to 4 carbon atoms, aminoalkyl of 1 to 4 carbon atoms, alkylaminoalkyl or dialkylaminoalkyl where alkyl is of 1 to 4 carbon atoms, or phenyl optionally mono- or disubstituted by chloro or fluoro or optionally monosubstituted by alkoxy of 1 to 4 carbon atoms or alkoxycarbonyl of 2 to 5 carbon atoms, and $R^3$ and $R^4$, independently of one another, are hydrogen, halogen or alkoxy of 1 to 4 carbon atoms,

or an acid addition salt of this compound.

4. A herbicide containing inert additives and a thiazolo-[2,3-b]-quinazolone of the formula I as claimed in claim 1, where $R^1$ and $R^2$, independently of one another, are hydrogen, alkyl or haloalkyl of 1 to 4 carbon atoms, aminoalkyl of 1 to 4 carbon atoms, alkylaminoalkyl or dialkylaminoalkyl where alkyl is of 1 to 4 carbon atoms, or phenyl optionally mono- or disubstituted by chloro or fluoro or optionally monosubstituted by alkoxy of 1 to 4 carbon atoms or alkoxycarbonyl of 2 to 5 carbon atoms, and $R^3$ and $R^4$, independently of one another, are hydrogen, halogen or alkoxy of 1 to 4 carbon atoms,

or an acid addition salt of this compound.

5. A herbicide containing a thiazolo[2,3-b]-quinazolone of the formula I as claimed in claim 1, where $R^1$ and $R^2$, independently of one another, are hydrogen, methyl, ethyl, bromomethyl, dialkylaminomethyl of 1 to 4 carbon atoms, or phenyl optionally mono- or disubstituted by chloro or fluoro or optionally monosubstituted by alkoxy of 1 to 4 carbon atoms, and $R^3$ and $R^4$, independently of one another, are hydrogen, chloro, bromo or methoxy,

or an acid addition salt of this compound.

6. A herbicide containing a thiazolo[2,3-b]-quinazolone of the formula I as claimed in claim 1, where $R^1$ is hydrogen, methyl, ethyl or bromomethyl, $R^2$ is hydrogen, methyl or ethyl, and $R^3$ and $R^4$, independently of one another, are hydrogen, chloro or bromo,

or an acid addition salt of this compound.

7. A herbicide containing inert additives and a thiazolo[2,3-b]-quinazolone of the formula I as claimed in claim 1, where $R^1$ is hydrogen, methyl, ethyl or bromomethyl, $R^2$ is hydrogen, methyl or ethyl, and $R^3$ and $R^4$, independently of one another, are hydrogen, chloro or bromo,

or an acid addition salt of this compound.

8. A herbicide containing a thiazolo[2,3-b]-quinazolone of the formula

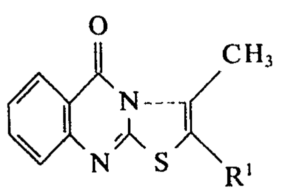

where
$R^1$ is hydrogen or methyl,
or an acid addition salt of this compound.

9. A herbicide containing inert additives and a thiazolo[2,3-]-quinazolone of the formula

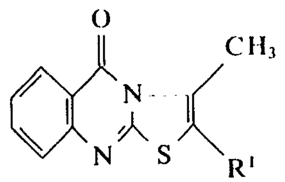

where
$R^1$ is hydrogen or methyl,
or an acid addition salt of this compound.

10. A process for combating unwanted plant growth and for influencing plant growth, wherein a herbicide as claimed in claim 1 is allowed to act on the plants and/or their location.

**Revendications**

1. Herbicide, contenant une thiazolo[2,3-b]-quinazolone de formule

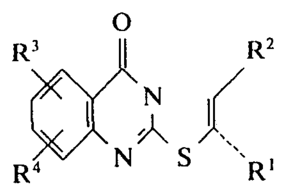

(I)

dans laquelle
$R^1$ et $R^2$ représentent, indépendamment l'un de l'autre, halogène, nitro, alkyle à 1 à 12 atomes de carbone, halogénalkyle à 1 à 4 atomes de carbone, cycloalkyle à 3 à 6 atomes de carbone, alcoxy ou alkylthio à 1 à 4 atomes de carbone, thiophényle, amino, alkylamino ou dialkylamino à 1 à 4 atomes de carbone dans un groupe alkyle, aminoalkyle à 1 à 4 atomes de carbone, alkylaminoalkyle ou dialkylaminoalkyle à 1 à 4 atomes de carbone dans un groupe alkyle, alcanoylamino ou halogénalcanoylamino à 2 à 5 atomes de carbone, dialkylcarbamido à 1 à 4 atomes de carbone dans un groupe alkyle, alcoxycarbonylalkyle à 3 à 6 atomes de carbone, alcoxycarbonyle à 2 à 6 atomes de carbone, éventuellement substitué par alcoxy à 1 à 4 atomes de carbone, sulfo, sulfino, alkylsulfonyle ou alkylsulfinyle à 1 à 4 atomes de carbone, chlorosulfonyle, phénylsulfonyle, sulfamoyle, alkylaminosulfinyle ou alkylaminosulfonyle à 1 à 4 atomes de carbone, hydroxyalkylaminosulfonyle ou di-hydroxyalkylaminosulfonyle à 1 à 4 atomes de carbone dans un groupe alkyle, morpholinylsulfonyle, alkylsulfonylamino à 1 à 4 atomes de carbone, alcoxycarbonylalkylamino à 3 à 6 atomes de carbone, phényle éventuellement substitué par halogène, une ou deux fois, par alcoxy ou carboxyalkyloxy ayant chacun jusqu'à 5 atomes de carbone, une fois, ou thien-2-yle, thiazol-2-yle, fur-2-yle, benzimidazol-2-yle, 2-chlorothien-4-yle, 2-méthyl-thien-4-yle ou 1-méthyl-benzimidazolyle éventuellement substitué par halogène ou alkyle à 1 à 4 atomes de carbone et
$R^3$ et $R^4$ représentent, indépendamment l'un de l'autre, hydrogène, halogène, nitro, alkyle ou halogénalkyle à 1 à 4 atomes de carbone, cycloalkyle à 3 à 6 atomes de carbone, alcoxy ou alkylthio à 1 à 4 atomes de carbone, thiophényle, amino, alkylamino ou dialkylamino à 1 à 4 atomes de carbone dans un groupe alkyle, aminoalkyle à 1 à 4 atomes de carbone, alkylaminoalkyle ou dialkylaminoalkyle à 1 à 4 atomes de carbone dans un groupe alkyle, alcanoylamino ou halogénalcanoylamino à 2 à 5 atomes de carbone, dialkylcarbamido à 1 à 4 atomes de carbone dans un groupe alkyle, alcoxycarbonylalkyle à 3 à 6 atomes de carbone, alcoxycarbonyle à 2 à 6 atomes de carbone éventuellement substitué par alcoxy à 1 à 4 atomes de carbone, sulfo, sulfino, alkylsulfonyle ou alkylsulfinyle à 1 à 4 atomes de carbone, chlorosulfonyle, phénylsulfonyle, sulfamoyle, alkylaminosulfinyle ou alkylaminosulfonyle à 1 à 4 atomes de carbone, hydroxyalkylaminosulfonyle ou dihydroxyalkylaminosulfonyle à 1 à 4 atomes de carbone dans un groupe alkyle, morpholinyl-sulfonyle, alkylsulfonylamino à 1 à 4 atomes de carbone, alcoxycarbonylalkylamino à 3 à 6 atomes de carbone, phényle éventuellement substitué par halogène, une ou deux fois, par alcoxy ou carboxyalkyloxy ayant chacun jusqu'à 5 atomes de carbone, une fois, ou thien-2-yle, thiazol-2-yle, fur-2-yle, benzimidazol-2-yle, 2-chloro-thien-4-yle, 2-méthyl-thien-4-yle ou 1-méthyl-benzimidazolyle éventuellement substitué une fois par halogène ou alkyle à 1 à 4 atomes de carbone,
ou des sels d'addition d'acide de ces composés.

2. Herbicide contenant des additifs inertes et une thiazolo[2,3-b]-quinazolone de formule

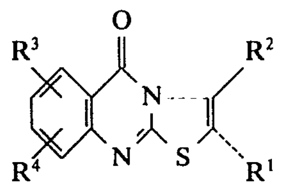

(I)

dans laquelle

$R^1$ et $R^2$ représentent, indépendamment l'un de l'autre, halogène, nitro, alkyle à 1 à 12 atomes de carbone, halogénalkyle à 1 à 4 atomes de carbone, cycloalkyle à 3 à 6 atomes de carbone, alcoxy ou alkylthio à 1 à 4 atomes de carbone, thiophényle, amino, alkylamino ou dialkylamino à 1 à 4 atomes de carbone dans un groupe alkyle, aminoalkyle à 1 à 4 atomes de carbone, alkylaminoalkyle ou dialkylaminoalkyle à 1 à 4 atomes de carbone dans un groupe alkyle, alcanoylamino ou halogénalcanoylamino à 2 à 5 atomes de carbone, dialkylcarbamido à 1 à 4 atomes de carbone dans un groupe alkyle, alcoxycarbonylalkyle à 3 à 6 atomes de carbone, alcoxycarbonyle à 2 à 6 atomes de carbone éventuellement substitué par alcoxy à 1 à 4 atomes de carbone, sulfo, sulfino, alkylsulfonyle ou alkylsulfinyle à 1 à 4 atomes de carbone, chlorosulfonyle, phénylsulfonyle, sulfamoyle, alkylaminosulfinyle ou alkylaminosulfonyle à 1 à 4 atomes de carbone, hydroxyalkylaminosulfonyle ou di-hydroxyalkylaminosulfonyle à 1 à 4 atomes de carbone dans un groupe alkyle, morpholinylsulfonyle, alkylsulfonylamino à 1 à 4 atomes de carbone, alcoxycarbonylalkylamino à 3 à 6 atomes de carbone, phényle éventuellement substitué par halogène, une ou deux fois, par alcoxy ou carboxyalkyloxy ayant chacun jusqu'à 5 atomes de carbone, une fois, ou thien-2-yle, thiazol-2-yle, fur-2-yle, benzimidazol-2-yle, 2-chlorothien-4-yle, 2-méthyl-thien-4-yle ou 1-méthyl-benzomidazolyle éventuellement substitué par halogène ou alkyle à 1 à 4 atomes de carbone et

$R^3$ et $R^4$ représentent, indépendamment l'un de l'autre, hydrogène, halogène, nitro, alkyle ou halogénalkyle à 1 à 4 atomes de carbone, cycloalkyle à 3 à 6 atomes de carbone, alcoxy ou alkylthio à 1 à 4 atomes de carbone, thiophényle, amino, alkylamino ou dialkylamino à 1 à 4 atomes de carbone dans un groupe alkyle, aminoalkyle à 1 à 4 atomes de carbone, alkylaminoalkyle ou dialkylaminoalkyle à 1 à 4 atomes de carbone dans un groupe alkyle, alcanoylamino ou halogénalcanoylamino à 2 à 5 atomes de carbone, dialkylcarbamido à 1 à 4 atomes de carbone dans un groupe alkyle, alcoxycarbonylalkyle à 3 à 6 atomes de carbone, alcoxycarbonyle à 2 à 6 atomes de carbone éventuellement substitué par alcoxy à 1 à 4 atomes de carbone, sulfo, sulfino, alkylsulfonyle ou alkylsulfinyle à 1 à 4 atomes de carbone, chlorosulfonyle, phénylsulfonyle, sulfamoyle, alkylaminosulfinyle ou alkylaminosulfonyle à 1 à 4 atomes de carbone, hydroxyalkylaminosulfonyle ou dihydroxyalkylaminosulfonyle à 1 à 4 atomes de carbone dans un groupe alkyle, morpholinyl-sulfonyle, alkylsulfonylamino à 1 à 4 atomes de carbone, alcoxycarbonylalkylamino à 3 à 6 atomes de carbone, phényle éventuellement substitué par halogène, une ou deux fois, par alcoxy ou carboxyalkyloxy ayant chacun jusqu'à 5 atomes de carbone, une fois, ou thien-2-yle, thiazol-2-yle, fur-2-yle, benzimidazol-2-yle, 2-chloro-thien-4-yle, 2-méthyl-thien-4-yle ou 1-méthyl-benzimidazolyle éventuellement substitué une fois par halogène ou alkyle à 1 à 4 atomes de carbone,

ou des sels d'addition d'acide de ces composés.

3. Herbicide contenant une thiazolo[2,3-b]-quinazolone de formule I, selon la revendication 1, où $R^1$ et $R^2$, indépendamment l'un de l'autre, représentent hydrogène, alkyle ou halogénalkyle à 1 à 4 atomes de carbone, aminoalkyle à 1 à 4 atomes de carbone, alkylaminoalkyle ou dialkylaminoalkyle à 1 à 4 atomes de carbone dans un groupe alkyle ou phényle, éventuellement substitué par fluor, une ou deux fois, par alcoxy à 1 à 4 atomes de carbone ou alcoxycarbonyle à 2 à 5 atomes de carbone, une fois, et $R^3$ et $R^4$ représentent, indépendamment l'un de l'autre, hydrogène, halogène, ou alcoxy à 1 à 4 atomes de carbone,

ou des sels d'addition d'acide de ces composés.

4. Herbicide contenant des additifs inertes et une thiazolo[2,3-b]-quinazolone de formule I, selon la revendication 1, où $R^1$ et $R^2$, indépendamment l'un de l'autre, représentent hydrogène, alkyle ou halogénalkyle à 1 à 4 atomes de carbone, aminoalkyle à 1 à 4 atomes de carbone, alkylaminoalkyle ou dialkylaminoalkyle à 1 à 4 atomes de carbone dans un groupe alkyle ou phényle, éventuellement substitué par fluor, une ou deux fois, par alcoxy à 1 à 4 atomes de carbone ou alcoxycarbonyle à 2 à 5 atomes de carbone, une fois, et $R^3$ et $R^4$ représentent, indépendamment l'un de l'autre, hydrogène, halogène, ou alcoxy à 1 à 4 atomes de carbone,

ou des sels d'addition d'acide de ces composés.

5. Herbicide contenant une thiazolo[2,3-b]-quinazolone de formule I selon la revendication 1, où $R^1$ et $R^2$, indépendamment l'un de l'autre, représentent hydrogène, méthyle, éthyle, bromométhyle, dialkylaminométhyle à 1 à 4 atomes de carbone ou phényle, éventuellement substitué, une ou deux

fois, par chlore ou fluor ou, une fois, par alcoxy à 1 à 4 atomes de carbone, et $R^3$ et $R^4$ représentent indépendamment l'un de l'autre, hydrogène, chlore, brome ou méthoxy,
ou les sels d'addition d'acide de ces composés.

6. Herbicide contenant une thiazolo[2,3-b]-quinazolone de formule I selon la revendication 1, où $R^1$ représente hydrogène, méthyle, éthyle ou bromométhyle, $R^2$ hydrogène, méthyle ou éthyle, et $R^3$ et $R^4$, indépendamment l'un de l'autre, hydrogène, chlore ou brome,
ou les sels d'addition d'acide de ces composés.

7. Herbicide contenant des additifs inertes et une thiazolo[2,3-b]-quinazolone de formule I selon la revendication 1, où $R^1$ représente hydrogène, méthyle, éthyle ou bromométhyle, $R^2$ hydrogène, méthyle ou éthyle, et $R^3$ et $R^4$, indépendamment l'un de l'autre, hydrogène, chlore ou brome,
ou les sels d'addition d'acide de ces composés.

8. Herbicide contenant une thiazolo[2,3-b]-quinazolone de formule

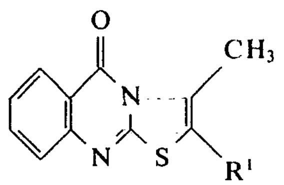

dans laquelle
$R^1$ représente hydrogène ou méthyle,
ou les sels d'addition d'acide de ces composés.

9. Herbicide contenant des additifs inertes et une thiazolo[2,3-b]-quinazolone de formule

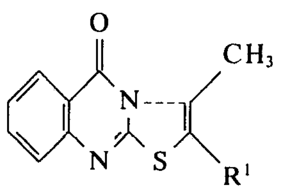

dans laquelle
$R^1$ représente hydrogène ou méthyle,
ou les sels d'addition d'acide de ces composés.

10. Procédé pour lutter contre la croissance des plantes indésirables et pour influer sur la croissance des plantes, caractérisé par le fait que l'on fait agir un herbicide selon la revendication 1 sur les plantes et/ou leur biotope.